# EUROPEAN PATENT SPECIFICATION

(11) **EP 4 225 291 B1**
(45) Date of publication and mention of the grant of the patent: **13.03.2024**
(21) Application number: 22704693.5
(22) Date of filing: 29.01.2022
(51) Int. Cl.: A61K 31/192, A61P 1/16, A61K 45/06

(54) **TREATMENT OF CHOLANGIOPATHIES WITH SELADELPAR**
BEHANDLUNG VON CHOLANGITIS MIT SELADELPAR
TRAITEMENT DE CHOLANGIOPATHIES AVEC SELADELPAR

(30) Priority: 01.02.2021 US 202163144355 P
(43) Date of publication of application: 16.08.2023
(73) Proprietor: CymaBay Therapeutics, Inc., Newark, CA 94560 (US)
(72) Inventor: CHOI, Yun-Jung, Newark, CA 94560 (US); MCWHERTER, Charles A., Newark, CA 94560 (US); STEINBERG, Alexandra S., Newark, CA 94560 (US); YANG, Ke, Newark, CA 94560 (US)
(74) Representative: Mewburn Ellis LLP
(86) International application number: PCT/US2022/014464
(87) International publication number: WO 2022/165288

(56) References cited:
- US-A1- 2017 340 589
- JONES DAVID ET AL: "Seladelpar (MBX-8025), a selective PPAR-Î agonist, in patients with primary biliary cholangitis with an inadequate response to ursodeoxycholic acid: a double-blind, randomised, placebo-controlled, phase 2, proof-of-concept study", THE LANCET - GASTROENTEROLOGY & HEPATOLOGY, ELSEVIER, US , vol. 2, no. 10 30 September 2017 (2017-09-30), pages 716-726, XP009530038, ISSN: 2468-1253, DOI: 10.1016/S2468-1253(17)30246-7 Retrieved from the Internet: URL:https://api.elsevier.com/content/artic le/PII:S2468125317302467?httpAccept=text/p lain [retrieved on 2017-08-14] cited in the application
- JORDAN GOLDSTEIN ET AL: "Novel and emerging therapies for cholestatic liver diseases", LIVER INTERNATIONAL, vol. 38, no. 9, 14 May 2018 (2018-05-14), pages 1520-1535, XP055723757, GB ISSN: 1478-3223, DOI: 10.1111/liv.13880
- MARINA G. SILVEIRA ET AL: "Investigational drugs in phase II clinical trials for primary biliary cholangitis", EXPERT OPINION ON INVESTIGATIONAL DRUGS, vol. 26, no. 10, 31 August 2017 (2017-08-31), pages 1115-1121, XP055523264, UK ISSN: 1354-3784, DOI: 10.1080/13543784.2017.1371135
- CICERO A F G ET AL: "Possible role of ubiquinone in the treatment of massive hypertriglyceridemia resistant to PUFA and fibrates", BIOMEDICINE & PHARMACOTHERAPY, ELSEVIER, FR, vol. 59, no. 6, 1 July 2005 (2005-07-01), pages 312-317, XP027640153, ISSN: 0753-3322 [retrieved on 2005-07-01]
- ISER J H ET AL: "Resistance to chenodeoxycholic acid (CDCA) treatment in obese patients with gall stones", BMJ. BRITISH MEDICAL JOURNAL, LONDON, GB, vol. 1, no. 6126, 10 June 1978 (1978-06-10), pages 1509-1512, XP009535135, ISSN: 0959-8146, DOI: 10.1136/BMJ.1.6126.1509

## Description

### Technical field

This invention relates to the treatment of cholangiopathies in subjects who are intolerant of, or have an inadequate response to, obeticholic acid and/or a fibrate.

### Background art

### Cholangiopathies

Cholangiocytes are the epithelial cells lining the intrahepatic and extrahepatic bile ducts, where they participate in bile production and homeostasis. In the healthy liver, cholangiocytes contribute to bile secretion via net release of bicarbonate and water. Cholangiocytes act through bile-acid independent bile flow, which is driven by the active transport of electrolytes. Cholangiocytes are damaged in a variety of human diseases called cholangiopathies. Cholestasis is a condition in which the flow of bile from the liver to the duodenum is slowed or blocked: in cholestasis, bile accumulates in the hepatic parenchyma. Cholestasis may be divided conveniently into two types: intrahepatic cholestasis, inside the liver, where bile formation is disturbed by conditions such as various diseases, extended intravenous nutrition, or as a side effect of certain drugs (such as some antibiotics); and extrahepatic cholestasis, occurring outside the liver, typically where the flow of bile is obstructed by a mechanical partial or complete closure of the bile duct, such as by bile duct tumors, cysts, bile duct stones, strictures, or pressure on the bile duct; though primary sclerosing cholangitis (PSC) may be intrahepatic or extrahepatic. Common symptoms of cholestasis include fatigue, pruritus (itching), jaundice, and xanthoma (deposits of cholesterol-rich material under the skin). The effects of cholestasis are profound and widespread, leading to worsening liver disease with systemic illness, liver failure, and the need for liver transplantation. As a group, cholangiopathies account for approximately 18% of adult liver transplantations and the majority of pediatric liver transplantations.

Intrahepatic cholangiopathies include, in order of decreasing frequency, primary biliary cholangitis (PBC, formerly known as primary biliary cirrhosis); primary sclerosing cholangitis (PSC) - which, as noted above, may also be extrahepatic; progressive familial intrahepatic cholestasis (PFIC); and Alagille syndrome (AS). Other cholangiopathies include cystic fibrosis associated cholangiopathy and the immune-mediated cholangiopathies autoimmune cholangitis and graft-versus-host disease involving the liver. Other cholangiopathies are mentioned in, for example, Box 1: Selected cholangiopathies at page 272 in Banales et al., "Cholangiocyte pathobiology", Nature Rev. Gastroenterol. Hepatol., vol. 16, pages 269-281 (2019), or other references on cholangiopathy.

PBC is an autoimmune condition of the liver marked by the slow progressive destruction of the small bile ducts of the liver, with the intralobular ducts affected early in the condition. When these ducts are damaged, bile builds up in the liver (cholestasis) and over time damages the tissue, which can lead to scarring, fibrosis and cirrhosis. Recent studies have shown that it may affect up to 1 in 3,000 - 4,000 people, with a sex ratio at least 9:1 female to male. There is no cure for PBC, and liver transplantation often becomes necessary; but medication such as ursodeoxycholic acid (UDCA, ursodiol) to reduce cholestasis and improve liver function, cholestyramine to absorb bile acids, modafinil for fatigue, and fat-soluble vitamins (vitamins A, D, E, and K, since reduced bile flow makes it difficult for these vitamins to be absorbed) may slow the progression to allow a normal lifespan and quality of life. UDCA is approved in the United States to treat PBC, but about 40% of patients are reported to have an inadequate response to UDCA and about 5% are reported to be intolerant to UDCA treatment. Japanese researchers have reported that the addition of bezafibrate, a peroxisome proliferator-activated receptor (PPAR) pan-agonist and pregnane X receptor agonist, to UDCA is helpful in treating patients who are refractory to UDCA monotherapy, improving serum biliary enzymes, cholesterol, and triglycerides; Korean researchers have reported the addition of fenofibrate or bezafibrate to UDCA; and the BEZURSO study added bezafibrate to UDCA. Obeticholic acid (OCA, 6α-ethylchenodeoxycholic acid, Intercept's OCALIVA), a semi-synthetic bile acid analog that is a highly potent farnesoid X receptor agonist, was approved in 2016 in the United States for the treatment of PBC, either in addition to UDCA or as sole treatment when UDCA is not tolerated. However, about 50% of patients are reported to have an inadequate response to OCA, and OCA is widely reported to exacerbate the pruritus that is one of the symptoms of PBC. From the OCALIVA prescribing information, severe pruritus was reported in 23% of patients in the OCALIVA 10 mg arm, 19% of patients in the OCALIVA titration arm, and 7% of patients in the placebo arm in a 12-month double-blind randomized controlled trial of 216 patients. Prospective, observational, multicenter studies have reported OCA discontinuation rates of 12% to 17% with a significant proportion of patients (45% to 71%) discontinuing due to treatment-induced pruritus.

PSC is a chronic cholestatic liver condition characterized by intra- or extrahepatic biliary duct inflammation and fibrosis, eventually leading to cirrhosis. The underlying cause of the inflammation is believed to be autoimmunity; and about three-fourths of patients with PSC have inflammatory bowel disease, usually ulcerative colitis, though this is reported to vary by country, as is the prevalence (generally reported at about 1 in 10,000) and sex ratio (generally reported as predominately male). Standard treatment includes UDCA, which has been shown to lower elevated liver enzyme numbers in people with PSC, but has not improved liver survival or overall survival; and also includes antipruritics, cholestyramine, fat-soluble vitamins, and antibiotics to treat infections (bacterial cholangitis). In a study reported in 2009, long-term high-dose UDCA therapy was associated with improvement in serum liver tests in PSC but did not improve survival and was associated with higher rates of serious adverse events. Fenofibrate alone has been tested in PSC (NCT01142323); and the addition of fenofibrate or bezafibrate to UDCA has been reported in patients with an inadequate response to UDCA. Liver transplantation is the only proven long-term treatment.

PFIC refers to a group of three types of autosomal recessive disorders of childhood associated with intrahepatic cholestasis: deficiency of familial intrahepatic cholestasis 1 (PFIC-1), deficiency of bile salt export pump (PFIC-2), and deficiency of multidrug resistance protein 3 (PFIC-3). They have a combined incidence of 1 in 50,000 - 100,000. The onset of the condition is usually before age 2, with PFIC-3 usually appearing earliest, but patients have been diagnosed with PFIC even into adolescence. Patients usually show cholestasis, jaundice, and failure to thrive; and intense pruritus is characteristic. Fat malabsorption and fat-soluble vitamin deficiency may appear. Biochemical markers include a normal γ-glutamyl transpeptidase (GGT) in PFIC-1 and PFIC-2, but a markedly elevated GGT in PFIC-3; while serum bile acid levels are greatly elevated; though serum cholesterol levels are typically not elevated, as is seen usually in cholestasis, because the condition is due to a transporter as opposed to an anatomical problem with biliary cells. The condition is typically progressive without liver transplantation, leading to liver failure and death in childhood; and hepatocellular carcinoma may develop in PFIC-2 at a very early age. Medication with UDCA is common; supplemented by fat-soluble vitamins, cholestyramine, and pancreatic enzymes in PFIC-1.

AS, also known as Alagille-Watson syndrome, syndromic bile duct paucity, and arteriohepatic dysplasia, is an autosomal dominant disorder associated with liver, heart, eye and skeletal abnormalities, as well as characteristic facial features; with an incidence of about 1 in 100,000. The liver abnormalities are narrowed and malformed bile ducts within the liver; and these result in obstruction of bile flow, causing cirrhosis (scarring) of the liver. AS is predominately caused by changes in the *Jagged1* gene, located on chromosome 20. In 3 - 5 % of cases, the entire gene is deleted (missing) from one copy of chromosome 20; in the remainder, there are changes or mutations in the *Jagged1* DNA sequence. In a very small number of cases, less than 1 percent, changes in another gene, *Notch2,* result in AS. In about one-third of the cases, the mutation is inherited; in about two-thirds, the mutation is new in that case. There is no cure for AS, though the severity of liver disease typically peaks by 3 to 5 years of age and often resolves by 7 to 8 years of age. In some people, the hepatic disease will progress to end-stage liver disease and may require liver transplantation; approximately 15 % of patients with AS require liver transplantation. A number of different medications, for example UDCA, have been used to improve bile flow and reduce itching, and many patients are given high doses of fat-soluble vitamins.

Cystic fibrosis associated cholangiopathy, more commonly known as cystic fibrosis liver disease or CFLD, is a complication of cystic fibrosis that has been reported to affect about 30% of cystic fibrosis patients and to be the third most frequent cause of death in patients with cystic fibrosis. CFLD may progress through hepatic steatosis, optionally accompanied by hepatitis, to focal biliary cirrhosis and multilobular cirrhosis. In some adult patients with CFLD, the symptoms resemble those of PSC. UDCA is a common treatment, as is supplementation with fat-soluble vitamins.

According to Heathcote, "Autoimmune cholangitis", Clinics Liver Dis., vol. 2(2), pages 303-311 (1998), "The term 'autoimmune cholangitis' describes patients whose chronic liver disease has a biliary pattern typical of primary biliary cirrhosis (PBC), yet is associated with non-organ-specific antibodies typical of autoimmune hepatitis. Some feel that this condition is a variant of classical type I autoimmune hepatitis; others consider it a variant of PBC because of the absence of serum mitochondrial antibodies." UDCA and immunosuppressive agents such as azathioprine or corticosteroids such as prednisolone have been suggested for treatment.

Graft-versus-host disease (GVHD) is a common complication following allogeneic hematopoietic cell transplantation (HCT) that typically manifests as injury to the skin, gastrointestinal mucosa, and liver. Chronic GVHD of the liver is more typically an indolent cholestatic syndrome associated with abnormalities in the skin, oral mucosa, and lacrimal glands that presents after day 100 after allogeneic HCT. The common treatment is immunosuppressive therapy.

Alkaline phosphatase (ALP) and GGT are key markers of cholestasis. While an elevation of one of them alone does not indicate cholestasis, and other parameters would be needed for confirmation, elevation in both ALP and GGT is indicative of cholestasis; and a decrease in both indicates improvement of cholestasis. Thus, ALP and GGT levels serve as biochemical markers for the presence of biliary pathophysiology present in intrahepatic cholangiopathies, and ALP level has been used as a primary outcome marker in clinical studies of intrahepatic cholangiopathies such as PBC (including in the studies leading to US approval of OCA). Other relevant markers may include biomarkers of biliary duct degeneration, such as CK19, miR-506, and others (*see,* for example, Baghdasaryan et al., "Inhibition of intestinal bile acid absorption improves cholestatic liver and bile duct injury in a mouse model of sclerosing cholangitis" J. Hepatology, vol. 64, pp. 674-681 (2016) and Erice et al., "MiRNA-506 promotes primary biliary cholangitis-like features in cholangiocytes and immune activation", Hepatology, vol. 67(4), pp. 1420-1440 (2018)), markers of liver damage such as aspartate aminotransferase (AST) and alanine aminotransferase (ALT), and markers of fibrosis such as Col1α1, since fibrosis is more commonly seen in extrahepatic cholangiopathies than in intrahepatic cholangiopathies. Clinical markers reflective of treatment may include the biomarkers mentioned above, lack of progression in fibrosis or progression to cirrhosis; reduction in blood-based fibrosis markers such as ELF, Pro-C3, and Pro-C5; and lack of liver-related adverse events such as cholangitis, ascites, variceal bleeding, or progression in MELD.

### Treatments for cholangiopathies

As mentioned above, UDCA is a common treatment for cholangiopathies, because of its action in reducing cholestasis and improving liver function. However, a Cochrane Review of UDCA in PBC in 2012 found that, although UDCA showed a reduction in biomarkers of liver pathology, jaundice, and ascites, there was no evidence in the medical literature for any benefit of UDCA on mortality or liver transplantation, while its use was associated with weight gain and costs. While UDCA is also used in other cholangiopathies, the only long-term treatment for many patients with cholangiopathies is liver transplantation.

Also, as mentioned above, OCA was approved in 2016 in the United States for the treatment of PBC, either in addition to UDCA or as sole treatment when UDCA is not tolerated. Fibrates such as fenofibrate and bezafibrate have also been used, like OCA either in addition to UDCA or as sole treatment when UDCA is not tolerated or provides an inadequate response. Other drugs, such as the PPARα/δ agonist elafibranor have been tested in PBC, and both fenofibrate and bezafibrate have also been tested in PSC. Other drugs for cholangiopathies, in particular such as for PFIC and AS, target the cholestatic pruritus associated with these diseases by inhibiting the apical sodium-bile acid transporter ASBT [also referred to as the ileal bile acid transporter (IBAT)]. These include odevixibat, approved as Albireo's BYL V A Y in the United States for the treatment of PFIC, and maralixabat, approved as Mirum's LIVMARLI in the United States for treatment of AS.

However, the current US labeling for OCALIVA contains a "black box" warning against use in PBC patients with decompensated cirrhosis (e.g., Child-Pugh Class B or C) or a prior decompensation event, or compensated cirrhosis with evidence of portal hypertension; while the current US labeling for fenofibrate (AbbVie's TRICOR) states that it is contraindicated in patients with active liver disease, including those with primary biliary cirrhosis and unexplained persistent liver function abnormalities. Bezafibrate is not approved in the US, but the current Canadian labeling for sustained-release bezafibrate (Allergan's BEZALIP SR) states that it is contraindicated in hepatic impairment, including primary biliary cirrhosis. Goldstein et al. ("Novel and emerging therapies for cholestatic liver diseases", Liver Int., vol 38, pp. 1520-1535 (2018)) discusses the role of bile acids in the pathophysiology of cholestatic liver diseases and therapies that are under investigation. Silveira et al. ("Investigational drugs in phase II clinical trials for primary biliary cholangitis" Expert Opin. Investig. Drugs, vol 26, pp 1115-1121 (2017) discusses therapies being investigated in phase II clinical trials for PBC. Document US2017/340589 discloses the use of seladelpar for treating primary biliary cholangitis in patients in general.

It would be desirable to develop pharmacological treatments for cholangiopathies in subjects who are intolerant of, or who have an inadequate response to, obeticholic acid and/or a fibrate.

### Seladelpar

Seladelpar (International Nonproprietary Name - INN) has the chemical name [4-({(*2R*)-2-ethoxy-3-[4-(trifluoromethyl)phenoxy]propyl}sulfanyl)-2-methylphenoxy]acetic acid [IUPAC name from WHO Recommended INN: List 77], and the code number MBX-8025. Seladelpar, and its synthesis, formulation, and use, are disclosed in, for example, US Patent No. 7301050 (compound 15 in Table 1, Example M, claim 49), US Patent No. 7635718 (compound 15 in Table 1, Example M), and US Patent No. 8106095 (compound 15 in Table 1, Example M, claim 14). Lysine (L-lysine) salts of seladelpar and related compounds are disclosed in US Patent No. 7709682 (seladelpar L-lysine salt throughout the Examples, crystalline forms claimed).

Seladelpar is an orally active, potent (2 nM) agonist of PPARδ. It is specific (>600-fold and >2500-fold compared with PPARα and PPARγ receptors). PPARδ activation stimulates fatty acid oxidation and utilization, improves plasma lipid and lipoprotein metabolism, glucose utilization, and mitochondrial respiration, and preserves stem cell homeostasis. According to US Patent No. 7301050, PPARδ agonists, such as seladelpar, are suggested to treat PPARδ-mediated conditions, including "diabetes, cardiovascular diseases, Metabolic X syndrome, hypercholesterolemia, hypo-high density lipoprotein (HDL)-cholesterolemia, hyper-low density lipoprotein (LDL)-cholesterolemia, dyslipidemia, atherosclerosis, and obesity", with dyslipidemia said to include hypertriglyceridemia and mixed hyperlipidemia.

US Patent No. 9486428 and PCT International Publication No. WO 2015/143178 disclose the treatment of intrahepatic cholestatic diseases, such as primary biliary cholangitis, primary sclerosing cholangitis, progressive familial intrahepatic cholestasis, and Alagille syndrome, with seladelpar and its salts; US Patent No. 10272058 and PCT International Publication No. WO 2017/209865 disclose the treatment of the same diseases with lower doses of seladelpar and its salts, such as 5 and 10 mg/day of seladelpar; and US Application Publication No. 2019/0105291 and PCT International Publication No. WO 2019/06373 disclose the treatment of cholestatic pruritus with seladelpar and its salts.

Seladelpar has been studied in primary biliary cholangitis (PBC), with results for 50 and 200 mg/day reported in Jones et al., "Seladelpar (MBX-8025), a selective PPAR-δ agonist, in patients with primary biliary cholangitis with an inadequate response to ursodeoxycholic acid: a double-blind, randomised, placebo-controlled, phase 2, proof-of-concept study", Lancet Gastroenterol. Hepatol., 2(10), 716-726 (2017), and for 2, 5, and 10 mg/day at The International Liver Congress^{™} hosted by the European Association for the Study of Liver Diseases (EASL) in Paris, France (April 11-15, 2018): in poster LBP-2 (Hirschfield et al., "Treatment Efficacy and Safety of Seladelpar, a Selective Peroxisome Proliferator-Activated Receptor Delta agonist, in Primary Biliary Cholangitis Patients: 12- and 26-Week Analyses of an Ongoing, International, Randomized, Dose Ranging Phase 2 Study"), and in poster THU-239 (Boudes et al., "Seladelpar's Mechanism of Action as a Potential Treatment for Primary Biliary Cholangitis and Non-Alcoholic Steatohepatitis"), both available at https://ir.cymabay.com/presentations; and in later presentations. In the Phase 2b study, seladelpar was found to produce statistically significant decreases in ALP and GGT, together with a statistically significant decrease in ALT and stable total bilirubin levels, over a 52-week period; while similar results were seen even in a subgroup with Child-Pugh A cirrhosis. Seladelpar has also been proposed for other cholangiopathies.

Summary of the invention The invention is defined by the claims. Any subject-matter falling outside the scope of the claims is provided for information purposes only.

The references to methods of treatment in the detailed description of the invention in this description are to be interpreted as references to the compounds, pharmaceutical compositions and medicaments of the present invention for use in a method for treatment of the human (or animal) body by therapy.

This invention relates to seladelpar for use in methods of treating cholangiopathies in subjects who are intolerant of, or who have an inadequate response to, obeticholic acid and/or a fibrate, by administration of seladelpar or a salt thereof.

In various aspects, this invention includes:
seladelpar or a salt thereof for use in treating cholangiopathies in subjects who are intolerant of, or who have an inadequate response to, obeticholic acid and/or a fibrate;
the use of seladelpar or a salt thereof for treating, or in the manufacture of a medicament for treating, cholangiopathies in subjects who are intolerant of, or who have an inadequate response to, obeticholic acid and/or a fibrate; and
pharmaceutical compositions or medicaments comprising seladelpar or a salt thereof for treating cholangiopathies in subjects who are intolerant of, or who have an inadequate response to, obeticholic acid and/or a fibrate.

In view of the demonstrated efficacy of seladelpar in treating primary biliary cholangitis in subjects who are intolerant of, or who have an inadequate response to, obeticholic acid and/or a fibrate, as seen in Example 1, and the common factors of cholangiopathies, seladelpar is expected to have activity in treating other cholangiopathies in subjects who are intolerant of, or who have an inadequate response to, obeticholic acid and/or a fibrate. This activity is considered especially advantageous in view of the warnings and contraindications against the use of these agents in cholangiopathies such as PBC. This activity is expected whether the subjects are naive to, or whether they are intolerant of, or have an inadequate response to, ursodeoxycholic acid.

Preferred embodiments of this invention are characterized by the features of Claims 1 to 20 of this application as filed.

### Detailed description

### Definitions

Cholangiopathies and their treatment are described in the sections entitled "Cholangiopathies" and "Treatments for cholangiopathies" in the Background art.

Seladelpar is described in the subsection entitled "Seladelpar" of the Background art.

Salts (for example, pharmaceutically acceptable salts) of seladelpar are included in this invention and are useful in the methods described in this application. These salts are preferably formed with pharmaceutically acceptable acids. See, for example, "Handbook of Pharmaceutically Acceptable Salts", Stahl and Wermuth, eds., Verlag Helvetica Chimica Acta, Zürich, Switzerland, for an extensive discussion of pharmaceutical salts, their selection, preparation, and use. Unless the context requires otherwise, any reference to seladelpar is a reference both to the compound and to its salts.

Because seladelpar contains a carboxyl group, it may form salts when the acidic proton present reacts with inorganic or organic bases. Typically, seladelpar is treated with an excess of an alkaline reagent, such as hydroxide, carbonate or alkoxide, containing an appropriate cation. Cations such as Na⁺, K⁺, Ca²⁺, Mg²⁺, and NH₄⁺ are examples of cations present in pharmaceutically acceptable salts. Suitable inorganic bases, therefore, include calcium hydroxide, potassium hydroxide, sodium carbonate and sodium hydroxide. Salts may also be prepared using organic bases, such as salts of primary, secondary and tertiary amines, substituted amines including naturally-occurring substituted amines, and cyclic amines, including isopropylamine, trimethylamine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, *N*-ethylpiperidine, and the like. Useful salts are expected to include the L-lysine salts; and, as noted in the "Seladelpar" subsection, seladelpar is currently formulated as its L-lysine dihydrate salt.

"Another anti-cholestatic agent" refers to an agent used for the treatment of a cholangiopathy that is neither seladelpar or a seladelpar salt, nor obeticholic acid nor a fibrate. Such agents include ursodeoxycholic acid (UDCA), as mentioned in the Background art.

The term "fibrate" includes both derivatives of fibric acid, such as gemfibrozil, clofibrate, fenofibrate, bezafibrate, clofibride, ciprofibrate, clinofibrate, ronifibrate, and simfibrate, and non-fibric acid derivative compounds that are PPARα agonists (including mixed agonists provided that they have significant PPARα agonism), such as elafibranor, lanifibranor, and saroglitazar; particularly fenofibrate, bezafibrate, and elafibranor.

"Intolerant of" or "intolerance of" or similar terms, with respect to obeticholic acid and/or a fibrate, means that the subject administered the obeticholic acid and/or the fibrate for the treatment of a cholangiopathy, experiences such side effects that the subject discontinues treatment with the obeticholic acid and/or the fibrate. Exemplary side effects that may cause a subject to be considered intolerant to obeticholic acid include liver-related adverse reactions, pruritus, and reduction in HDL-C, for example as described in the current labeling for OCALIVA. Exemplary side effects that may cause a subject to be considered intolerant to a fibrate include muscle toxicity/myalgia, elevation in creatinine kinas, rhabdomyolysis, renal toxicity (such as elevation in serum creatinine or blood urea nitrogen, decline in glomerular filtration rate, elevation in ALT, AST or total bilirubin (TBIL)). "Obeticholic acid and/or a fibrate" and "at least one of obeticholic acid and a fibrate" refer to all of obeticholic acid, a single fibrate, more than one fibrate, obeticholic acid and a single fibrate, and obeticholic acid and more than one fibrate.

An "inadequate response", "incomplete response", or similar terms, with respect to obeticholic acid and/or a fibrate, means that the subject administered the obeticholic acid and/or a fibrate for the treatment of a cholangiopathy, fails to experience adequate treatment of their cholangiopathy. An inadequate response may be assessed by a failure of the subject to achieve an adequate lowering of biochemical markers of the cholangiopathy, such as by a failure of the subject to achieve at least one of, and generally more than one of, an ALP less than 1.67× the upper limit of normal, a sufficient (e.g., at least 15%) decrease in ALP, and/or a total bilirubin less than the upper limit of normal.

Because ursodeoxycholic acid is regarded as first-line therapy for many cholangiopathies, other therapies, such as obeticholic acid, are typically required to be tested in subjects who are intolerant of, or have an inadequate response to, ursodeoxycholic acid; or are tested as an add-on to ursodeoxycholic acid, such as in the BEZURSO and POISE studies. Thus, experience in subjects who are naive to, i.e., untreated with, ursodeoxycholic acid is essentially unavailable. However, seladelpar is expected to be as effective in subjects who are naive to ursodeoxycholic acid as well as in subjects who are intolerant of, or have an inadequate response to, ursodeoxycholic acid.

"Concomitant administration" of seladelpar and another anti-cholestatic agent, such as ursodeoxycholic acid) means administration of the seladelpar and the another anti-cholestatic agent during the course of treatment of a cholangiopathy. Such concomitant administration may involve administration of the another anti-cholestatic agent before, during, and/or after administration of the seladelpar, such that therapeutically effective levels of each of the compounds are maintained. Concomitant administration may be accomplished by administration of the seladelpar and the another anti-cholestatic agent each at its usual dosing; but if both are orally bioavailable and conveniently have daily oral dosing, concomitant administration might include also administration of a combination dosage form. "Combination therapy" with seladelpar and another anti-cholestatic agent has the same meaning as "concomitant administration".

A "therapeutically effective amount" of seladelpar or a salt thereof means that amount which, when administered for the treatment of a cholangiopathy in a subject (i.e. a human) who is intolerant of, or who has an inadequate response to, obeticholic acid and/or a fibrate, is sufficient to effect treatment for the cholangiopathy.
"Treating" or "treatment" of a cholangiopathy in a subject includes one or more of:
(1) preventing or reducing the risk of developing a cholangiopathy, i.e., causing the clinical symptoms of the cholangiopathy not to develop in a subject who may be predisposed to an cholangiopathy but who does not yet experience or display symptoms of the cholangiopathy (i.e. prophylaxis);
(2) inhibiting a cholangiopathy, i.e., arresting or reducing the development of the cholangiopathy or its clinical symptoms; and
(3) relieving a cholangiopathy, i.e., causing regression, reversal, or amelioration of the cholangiopathy or reducing the number, frequency, duration or severity of its clinical symptoms.

"Treatment" does not necessarily imply "cure" or complete treatment, e.g. treatment of all clinical symptoms of a cholangiopathy, though "treatment" may include "cure". Rather, "treatment" implies the provision of clinical benefit by the administration of seladelpar when compared to non-administration of seladelpar; and treatment may also be assessed by improvement in biological markers of the cholangiopathy being treated.

The therapeutically effective amount for a particular subject varies depending upon the health and physical condition of the subject to be treated, the extent of the cholangiopathy, the assessment of the medical situation, and other relevant factors. It is expected that the therapeutically effective amount will fall in a relatively broad range that can be determined through routine trial.

If the seladelpar is administered concomitantly administered with another anti-cholestatic agent that is not obeticholic acid nor a fibrate, then a "therapeutically effective amount" of seladelpar, or of the another anti-cholestatic agent administered concomitantly with the seladelpar, means that amount of each which, when the seladelpar and the another anti-cholestatic agent are concomitantly administered to a subject for treating a cholangiopathy, is sufficient to effect treatment for the cholangiopathy.

"Comprising" or "containing" and their grammatical variants are words of inclusion and not of limitation and mean to specify the presence of stated components, groups, steps, and the like but not to exclude the presence or addition of other components, groups, steps, and the like. Thus "comprising" does not mean "consisting of" "consisting substantially of" or "consisting only of"; and, for example, a formulation "comprising" a compound must contain that compound but also may contain other active ingredients and/or excipients. Unless the context requires otherwise, singular forms "a," "an," and "the" include plural referents. Thus, for example, "the another anti-cholestatic agent" indicates one or more another anti-anticholestatic agents; and "a compound selected from seladelpar and the salts thereof' indicates one or more compounds chosen from the group consisting of seladelpar and salts of seladelpar.

### Formulation and administration

Seladelpar may be administered by any route suitable to the subject being treated and the nature of the subject's condition. Routes of administration include oral administration (generally preferred, if available); administration by injection, including intravenous, intraperitoneal, intramuscular, and subcutaneous injection; by transmucosal (e.g., intranasal, buccal, sublingual, rectal, or vaginal) or transdermal (topical) delivery; and the like. Formulations may be oral formulations (e.g., tablets, capsules, or oral solutions or suspensions); injectable formulations (e.g., solutions); and formulations designed to administer the drug across mucosal membranes or transdermally. Suitable formulations for each of these methods of administration may be found, for example, in "Remington: The Science and Practice of Pharmacy", 20th ed., Gennaro, ed., Lippincott Williams & Wilkins, Philadelphia, Pa., U.S.A. Because seladelpar is orally available, typical formulations will be oral, and typical dosage forms will be tablets or capsules for oral administration. As mentioned in the "Seladelpar" subsection, seladelpar has been formulated in capsules for clinical trials. Intravenous formulations may be particularly applicable for administration to acutely ill subjects, such as subjects suffering from acute alcoholic hepatitis or alcoholic fibrosis or cirrhosis, such as those subjects who may be hospitalized for treatment.

Depending on the intended mode of administration, the pharmaceutical compositions may be in the form of solid, semi-solid or liquid dosage forms, preferably in unit dosage form suitable for single administration of a precise dosage. In addition to an effective amount of seladelpar, the compositions may contain suitable pharmaceutically-acceptable excipients, including adjuvants which facilitate processing of the active compounds into preparations which can be used pharmaceutically. "Pharmaceutically acceptable excipient" refers to an excipient or mixture of excipients which does not interfere with the effectiveness of the biological activity of the active compound(s) and which is not toxic or otherwise undesirable to the subject to which it is administered.

For solid compositions, conventional excipients include, for example, pharmaceutical grades of mannitol, lactose, starch, magnesium stearate, sodium saccharin, talc, cellulose, glucose, sucrose, magnesium carbonate, and the like. Liquid pharmacologically administrable compositions can, for example, be prepared by dissolving, dispersing, etc., an active compound as described herein and optional pharmaceutical adjuvants in water or an aqueous excipient, such as, for example, water, saline, aqueous dextrose, and the like, to form a solution or suspension. If desired, the pharmaceutical composition to be administered may also contain minor amounts of nontoxic auxiliary excipients such as wetting or emulsifying agents, pH buffering agents and the like, for example, sodium acetate, sorbitan monolaurate, triethanolamine sodium acetate, triethanolamine oleate, etc.

For oral administration, the composition will generally take the form of a tablet or capsule; or, especially for pediatric use, it may be an aqueous or nonaqueous solution, suspension or syrup. Tablets and capsules are preferred oral administration forms. Tablets and capsules for oral use will generally include one or more commonly used excipients such as lactose and corn starch. Lubricating agents, such as magnesium stearate, are also typically added. When liquid suspensions are used, the active agent may be combined with emulsifying and suspending excipients. If desired, flavoring, coloring and/or sweetening agents may be added as well. Other optional excipients for incorporation into an oral formulation include preservatives, suspending agents, thickening agents, and the like.

Typically, a pharmaceutical composition of seladelpar, or a kit comprising compositions of seladelpar, is packaged in a container with a label, or instructions, or both, indicating use of the pharmaceutical composition or kit in the treatment of alcoholic liver disease.

A person of ordinary skill in the art of pharmaceutical formulation will be able to prepare suitable pharmaceutical compositions of the seladelpar by choosing suitable dosage forms, excipients, packaging, and the like, to achieve therapeutically effective formulations without undue experimentation and in reliance upon personal knowledge and the disclosure of this application.

A suitable (i.e. a therapeutically effective) amount of seladelpar or a salt thereof for oral dosing is expected to be equivalent to at least 0.5 mg/day, for example at least 1 mg/day, such as at least 2 mg/day, or at least 5 mg/day of seladelpar; but equivalent to not more than 50 mg/day, for example not more than 25 mg/day, such as not more than 15 mg/day, or not more than 10 mg/day of seladelpar; for example within any range defined by one of the "at least" values and one of the "not more than" values, such as at least 1 mg/day and not more than 25 mg/day (i.e. 1 - 25 mg/day) or at least 2 mg/day and not more than 10 mg/day; for example 2 mg/day, 5 mg/day, or 10 mg/day, for an adult subject who is intolerant of, or who has an inadequate response to, obeticholic acid and/or a fibrate, depending on the extent and severity of the cholangiopathy and factors such as hepatic and renal function. That is, a suitable amount of seladelpar for oral dosing for adults to treat conditions such as PBC is expected to be about the same as for a similar subject who is not intolerant of, or who has an adequate response to, obeticholic acid and/or a fibrate. Suitable reductions in dose toward or below the lower end of the outer range above will be made for subjects who are children in diseases such as PFIC and AS, depending on such additional factors as age and body mass; and in subjects with significant hepatic impairment, such as subjects in Child-Pugh classes B and C, depending on the degree of impairment. These amounts represent an average daily dose, and not necessarily an amount given at a single dose. Dosing may be as frequent as more than once/day (where the amount, or daily dose, will be divided between the number of administrations per day), but will more typically be once/day (where the amount is given in a single administration). Optionally, particularly in cases of significant hepatic impairment, the dosing may be less frequent than once/day, such as between once/week and every other day, for example once/week, twice/week (especially with the doses at least three days apart), three times/week (especially with the doses at least two days apart), or every other day; so that, as an example, a subject may receive 5 mg twice/week for an amount (daily dose) of 1.4 mg/day. An amount of a seladelpar salt that is "equivalent to" a particular amount of seladelpar refers to that amount of the salt that is the particular amount multiplied by the ratio of the formula weight of the salt to the formula weight of seladelpar. For example, if seladelpar L-lysine dihydrate salt is being used, since the formula weight of seladelpar L-lysine dihydrate salt is about 1.41 times the formula weight of seladelpar, an amount of about 14.1 mg/day of seladelpar L-lysine dihydrate salt will be equivalent to an amount of 10 mg/day of seladelpar.

When seladelpar or a seladelpar salt and another anti-cholestatic agent are concomitantly administered, a suitable amount of the seladelpar or a seladelpar salt is expected to be the same as when the seladelpar or a seladelpar salt is administered alone; and a suitable amount of the another anti-cholestatic agent is expected to be similar to the amount approved or used in clinical trials, as described in the Background art. That is, suitable amounts of the seladelpar or a seladelpar salt and the another anti-cholestatic agent to achieve a therapeutically effective amount of the combination therapy will be similar to the amounts employed in clinical trials. However, it is possible that the therapeutically effective amounts of either may be less in combination therapy than when used as monotherapy because each of them is expected to be effective in treating a cholangiopathy.

A person of ordinary skill in the art of the treatment of cholangiopathies will be able to ascertain a therapeutically effective amount of the seladelpar or a seladelpar salt, when used alone or concomitantly with another anti-cholestatic agent, and the another anti-cholestatic agent when used in concomitant administration, for a particular patient and stage of the cholangiopathy, to achieve a therapeutically effective amount without undue experimentation and in reliance upon personal knowledge and the disclosure of this application.

### Examples

### Example 1: Primary biliary cholangitis

Subjects with primary biliary cholangitis who were enrolled in an open-label Phase 2 study (NCT02955602, EudraCT 2016-002996-91) or a randomized and placebo-controlled Phase 3 study (NCT03602560, EudraCT 2018-001171-20) of seladelpar in subjects who were intolerant of, or had an inadequate response to, ursodeoxycholic acid, were pooled; and subjects who were intolerant of, or had an inadequate response to, obeticholic acid and/or a fibrate, were selected for analysis. The trial subjects were adult, male or female, with a diagnosis of PBC by at least two of the following three criteria: (a) a history of ALP above the upper limit of normal (ULN) for at least six months, (b) positive anti-mitochondrial antibody titers > 1/40 on immunofluorescence or M2 positive by enzyme linked immunosorbent assay or positive PBC-specific antinuclear antibodies, and (c) documented liver biopsy result consistent with PBC, on a stable and recommended dose of UDCA for the past twelve months or UDCA intolerant, and ALP ≥ 1.67 × ULN. Exclusion criteria included AST or ALT ≥ 3 × ULN, TBIL ≥ 2 × ULN, autoimmune hepatitis or a history of chronic viral hepatitis, PSC, the current use of fibrates or simvastatin, the use of colchicine, methotrexate, azathioprine, or systemic steroids in the previous two months, the use of an experimental treatment for PBC, and the use of an experimental or unapproved immunosuppressant. Subjects received seladelpar L-lysine dihydrate salt in an amount equivalent to either 10 mg/day or 5 mg/day of seladelpar (S), or placebo, orally once/day in capsule form. Efficacy was assessed on subjects treated for three months. The composite endpoint was the responder rate for subjects achieving ALP < 1.67 × ULN, > 15% decrease in ALP, and TBIL ≤ ULN. Additional endpoints were ALP ≤ ULN, ALP change from baseline, and other markers of liver function. Safety was assessed over one year.

A total of 71 subjects of the 384 enrolled subjects in the two studies were intolerant of, or had an inadequate response to, OCA (47), a fibrate (16), or both (8). Fifty-one subjects were treated for 3 months; and 37 were assessed; with results in the table below:

| | **S 10 mg/day** | **S 5 mg/day** | **Placebo** |
|---|---|---|---|
| Number of subjects | 12 | 13 | 12 |
| Mean baseline ALP (U/L) | 307 | 345 | 334 |
| Mean baseline ALT (U/L) | 48 | 55 | 48 |
| Mean baseline TBIL (mg/dL) | 0.77 | 0.85 | 0.71 |
| Composite endpoint met | 79% | 40% | 8% |
| ALP ≤ ULN | 21% | 0 | 0 |
| Mean change in ALP | -45% | -31% | -9% |
| Mean change in ALT | -21% | -13% | -7% |
| Mean change in TBIL | -9% | -3% | +3% |
| Pruritus adverse events experienced | 7% | 15% | 18% |

A safety analysis was performed on all 71 subjects. Four subjects experienced a serious adverse event (three on seladelpar 5 mg/day and one on placebo), all unrelated to seladelpar; two subjects discontinued treatment due to adverse events: one for ALT/AST elevation, and one for gastroesophageal reflux disease, both on seladelpar 5 mg/day. In subjects with PBC who are intolerant of, or had an inadequate response to, obeticholic acid and/or a fibrate, seladelpar appeared to be safe, well tolerated, and showed meaningful improvement in biochemical markers of cholestasis.

### Example 2: Primary sclerosing cholangitis

Trial subjects are adult, male or female, with a diagnosis of PSC by at least two of the following three criteria: (a) historical evidence of an elevated AP > ULN from any prior laboratory result, (b) liver biopsy consistent with PSC, and (c) abnormal cholangiography consistent with PSC as measured by MRCP, ERCP, or percutaneous transhepatic cholangiography; on a stable and recommended dose of UDCA ≤ 20 mg/Kg/day for the past six months or at least twelve weeks off UDCA treatment; and intolerant of, or having an inadequate response to, obeticholic acid and/or a fibrate. Other criteria include ALP ≥ 1.5 × ULN, TBIL ≤ 2 × ULN, ALT and AST both ≤ 5 × ULN, eGFR > 60 mL/min/1.73 m², platelets ≥ 140 × 10³/µL, INR ≤ 1.3 (in the absence of warfarin or other anticoagulant therapy), and albumin ≥ 3.5 g/dL. Exclusion criteria include clinically significant acute or chronic liver disease of an etiology other than PSC, a diagnosis of overlapping autoimmune hepatitis (AIH) and PSC, secondary or IgG4 related sclerosing cholangitis, small duct PSC, presence of a cholangiocarcinoma on cholangiography or MRI, bile duct stenting, history, evidence, or high suspicion of cholangiocarcinoma or other hepatobiliary malignancy, presumptive or diagnosed acute cholangitis within twelve weeks, and evidence of compensated or decompensated cirrhosis. The primary study endpoint is relative change in baseline serum ALP at week 24; and secondary endpoints are the incidence of treatment-emergent adverse events, the incidence and severity of PSC-related symptoms or procedures, and the incidence of hepatic disease progression events. Subjects are randomized to receive either placebo, or seladelpar or a salt thereof in an amount equivalent to 5, 10, or 20 mg/day of seladelpar, orally once/day for 24 weeks. The subjects show dose-related reductions in ALP, and demonstrate improvements in PSC-related symptoms.

## Claims

1. A compound selected from seladelpar and the salts thereof for use in treating a cholangiopathy in a subject who is intolerant of, or who has an inadequate response to, at least one of obeticholic acid and a fibrate.

2. The compound for use of claim 1 where the compound is a seladelpar L-lysine salt.

3. The compound for use of claim 2 where the compound is seladelpar L-lysine dihydrate salt.

4. The compound for use of any one of claims 1 to 3 where the compound is administered orally.

5. The compound for use of any one of claims 1 to 4 where the amount of the compound is equivalent to 0.5 - 50 mg/day of seladelpar.

6. The compound for use of claim 5 where the amount of the compound is equivalent to 1 - 25 mg/day of seladelpar.

7. The compound for use of claim 6 where the amount of the compound is equivalent to 2 - 10 mg/day of seladelpar.

8. The compound for use of claim 7 where the amount of the compound is equivalent to 5 mg/day or 10 mg/day of seladelpar.

9. The compound for use of any one of claims 1 to 8 where the compound is administered once/day.

10. The compound for use of any one of claims 1 to 9 where the cholangiopathy is primary biliary cholangitis.

11. The compound for use of any one of claims 1 to 9 where the cholangiopathy is primary sclerosing cholangitis.

12. The compound for use of any one of claims 1 to 9 where the cholangiopathy is progressive familial intrahepatic cholestasis.

13. The compound for use of any one of claims 1 to 9 where the cholangiopathy is Alagille syndrome.

14. The compound for use of any one of claims 1 to 9 where the cholangiopathy is cystic fibrosis associated cholangiopathy.

15. The compound for use of any one of claims 1 to 9 where the cholangiopathy is autoimmune cholangitis.

16. The compound for use of any one of claims 1 to 9 where the cholangiopathy is graft-versus-host disease involving the liver.

17. The compound for use of any one of claims 1 to 16 where the subject is naive to ursodeoxycholic acid.

18. The compound for use of any one of claims 1 to 16 where the subject is intolerant of, or has an inadequate response to, ursodeoxycholic acid.

19. The compound for use of any one of claims 1 to 18 where the subject is intolerant of, or has an inadequate response to, obeticholic acid.

20. The compound for use of any one of claims 1 to 19 where the subject is intolerant of, or has an inadequate response to a fibrate.

## Patentansprüche

1. Verbindung, ausgewählt aus Seladelpar und den Salzen davon, zur Verwendung bei der Behandlung einer Cholangiopathie in einem Individuum, das gegenüber zumindest einem aus Obeticholsäure und einem Fibrat intolerant ist oder eine nicht angemessene Reaktion darauf aufweist.

2. Verbindung zur Verwendung nach Anspruch 1, wobei die Verbindung ein Seladelpar-L-Lysinsalz ist.

3. Verbindung zur Verwendung nach Anspruch 2, wobei die Verbindung ein Seladelpar-L-Lysindihydratsalz ist.

4. Verbindung zur Verwendung nach einem der Ansprüche 1 bis 3, wobei die Verbindung oral verabreicht wird.

5. Verbindung zur Verwendung nach einem der Ansprüche 1 bis 4, wobei die Menge der Verbindung gleich 0,5 bis 50 mg/Tag Seladelpar beträgt.

6. Verbindung zur Verwendung nach Anspruch 5, wobei die Menge der Verbindung gleich 1 bis 25 mg/Tag Seladelpar beträgt.

7. Verbindung zur Verwendung nach Anspruch 6, wobei die Menge der Verbindung gleich 2 bis 10 mg/Tag Seladelpar beträgt.

8. Verbindung zur Verwendung nach Anspruch 7, wobei die Menge der Verbindung gleich 5 mg/Tag oder 10 mg/Tag Seladelpar beträgt.

9. Verbindung zur Verwendung nach einem der Ansprüche 1 bis 8, wobei die Verbindung einmal täglich verabreicht wird.

10. Verbindung zur Verwendung nach einem der Ansprüche 1 bis 9, wobei die Cholangiopathie primäre biliäre Cholangitis ist.

11. Verbindung zur Verwendung nach einem der Ansprüche 1 bis 9, wobei die Cholangiopathie primäre sklerosierende Cholangitis ist.

12. Verbindung zur Verwendung nach einem der Ansprüche 1 bis 9, wobei die Cholangiopathie progressive familiäre intrahepatische Cholestase ist.

13. Verbindung zur Verwendung nach einem der Ansprüche 1 bis 9, wobei die Cholangiophathie Alagille-Syndrom ist.

14. Verbindung zur Verwendung nach einem der Ansprüche 1 bis 9, wobei die Cholangiopathie mit zystischer Fibrose in Zusammenhang stehende Cholangiopathie ist.

15. Verbindung zur Verwendung nach einem der Ansprüche 1 bis 9, wobei die Cholangiopathie Autoimmun-Cholangitis ist.

16. Verbindung zur Verwendung nach einem der Ansprüche 1 bis 9, wobei die Cholangiopathie Graft-versus-Host-Erkrankung mit Leberbeteiligung ist.

17. Verbindung zur Verwendung nach einem der Ansprüche 1 bis 16, wobei das Individuum naiv gegenüber Ursodesoxycholsäure ist

18. Verbindung zur Verwendung nach einem der Ansprüche 1 bis 16, wobei das Individuum gegenüber Ursodesoxycholsäure intolerant ist oder eine nicht angemessene Reaktion darauf aufweist.

19. Verbindung zur Verwendung nach einem der Ansprüche 1 bis 18, wobei das Individuum gegenüber Obeticholsäure intolerant ist oder eine nicht angemessene Reaktion darauf aufweist.

20. Verbindung zur Verwendung nach einem der Ansprüche 1 bis 19, wobei das Individuum gegenüber einem Fibrat intolerant ist oder eine nicht angemessene Reaktion darauf aufweist.

## Revendications

1. Composé choisi parmi le séladelpar et ses sels à utiliser dans le traitement d'une cholangiopathie chez un sujet qui est intolérant à, ou qui présente une réponse inadéquate à, au moins un parmi l'acide obéticholique et un fibrate.

2. Composé à utiliser selon la revendication 1, dans lequel le composé est un sel de L-lysine de séladelpar.

3. Composé à utiliser selon la revendication 2, dans lequel le composé est le sel dihydrate de L-lysine de séladelpar.

4. Composé à utiliser selon l'une quelconque des revendications 1 à 3, dans lequel le composé est administré par voie orale.

5. Composé à utiliser selon l'une quelconque des revendications 1 à 4, dans lequel la quantité du composé équivaut à 0,5 à 50 mg/jour de séladelpar.

6. Composé à utiliser selon la revendication 5, dans lequel la quantité du composé équivaut à 1 à 25 mg/jour de séladelpar.

7. Composé à utiliser selon la revendication 6, dans lequel la quantité du composé équivaut à 2 à 10 mg/jour de séladelpar.

8. Composé à utiliser selon la revendication 7, dans lequel la quantité du composé équivaut à 5 mg/jour ou 10 mg/jour de séladelpar.

9. Composé à utiliser selon l'une quelconque des revendications 1 à 8, dans lequel le composé est administré une fois par jour.

10. Composé à utiliser selon l'une quelconque des revendications 1 à 9, dans lequel la cholangiopathie est une cholangite biliaire primitive.

11. Composé à utiliser selon l'une quelconque des revendications 1 à 9, dans lequel la cholangiopathie est une cholangite sclérosante primitive.

12. Composé à utiliser selon l'une quelconque des revendications 1 à 9, dans lequel la cholangiopathie est une cholestase intrahépatique familiale progressive.

13. Composé à utiliser selon l'une quelconque des revendications 1 à 9, dans lequel la cholangiopathie est le syndrome d'Alagille.

14. Composé à utiliser selon l'une quelconque des revendications 1 à 9, dans lequel la cholangiopathie est une cholangiopathie associée à la mucoviscidose.

15. Composé à utiliser selon l'une quelconque des revendications 1 à 9, dans lequel la cholangiopathie est une cholangite auto-immune.

16. Composé à utiliser selon l'une quelconque des revendications 1 à 9, dans lequel la cholangiopathie est une maladie du greffon contre l'hôte impliquant le foie.

17. Composé à utiliser selon l'une quelconque des revendications 1 à 16, dans lequel le sujet est innocent par rapport à l'acide ursodésoxycholique.

18. Composé à utiliser selon l'une quelconque des revendications 1 à 16, dans lequel le sujet est intolérant ou présente une réponse inadéquate à l'acide ursodésoxycholique.

19. Composé à utiliser selon l'une quelconque des revendications 1 à 18, dans lequel le sujet est intolérant ou présente une réponse inadéquate à l'acide obéticholique.

20. Composé à utiliser selon l'une quelconque des revendications 1 à 19, dans lequel le sujet est intolérant ou présente une réponse inadéquate à un fibrate.
